# EUROPEAN PATENT APPLICATION

(11) **EP 4 632 381 A1**
(43) Date of publication of application: **15.10.2025**
(21) Application number: 24744704.8
(22) Date of filing: 18.01.2024
(51) Int. Cl.: G01N 33/53, C12Q 1/68, C12Q 1/6844, C12Q 1/6876, G01N 33/569, G01N 33/574

(54) **METHOD AND KIT FOR PREDICTING EFFICACY OF TREATMENT WITH IMMUNOTHERAPEUTIC AGENT ON PATIENT WITH MUCOSAL LESION**

(30) Priority: 18.01.2023 JP 2023005795
(71) Applicant: Nihon University, Tokyo 102-8275 (JP); GLOVACC Co., Ltd., Tokyo 105-0011 (JP)
(72) Inventor: KAWANA Kei, Tokyo 102-8275 (JP); KATOH Yuki, Tokyo 102-8275 (JP); SEWAKI Tomomitsu, Tokyo 105-0011 (JP)
(74) Representative: Potter Clarkson
(86) International application number: PCT/JP2024/001302
(87) International publication number: WO 2024/154784

(57) **Abstract**

The present invention provides a novel method and a novel kit for predicting the efficacy of a treatment with immunotherapeutic agent on a patient with mucosal lesion. The present invention is a method for predicting the efficacy of a treatment with immunotherapeutic agent on a patient with mucosal lesion, the method including a step of measuring an expression level of CD86 in a biological sample derived from the patient with mucosal lesion. The present invention is a kit for use in the method, the kit including a substance that specifically binds to CD86, or a forward primer and a reverse primer for amplifying a nucleic acid that encodes CD86.

## Description

### TECHNICAL FIELD

The present invention relates to a method and a kit for predicting the efficacy of a treatment with an immunotherapeutic agent on a patient with mucosal lesion.

Priority is claimed on Japanese Patent Application No. 2023-005795, filed on January 18, 2023, the content of which is incorporated herein by reference.

### BACKGROUND ART

Mucosal lesions are diseases that occur on the mucosa, and human papillomavirus (HPV) infection is one of the diseases. HPVs are DNA viruses, and to date, 100 or more different types thereof have been reported. HPVs infect the skin (for example, HPV1 and HPV2) or the mucosal surface (for example, HPV6 and HPV11), and are present at the infected site for several months to several years, thereby forming benign tumors (warts). Furthermore, among the HPVs, 15 types of HPVs (for example, HPV16, HPV18, HPV31, HPV33, HPV52, and HPV58) infect the mucosa (cervix, anus, oropharynx, oral cavity, vagina, vulva, penis, and the like) for a long period of time, cause tumorous lesions, and lead to the development of cancers in various organs (HPV-related tumors) afterwards. Thus, these are referred to as high-risk HPVs. Among the high-risk HPVs, the HPV16 type has the highest malignancy, and about 60% of cervical cancers and about 90% of other HPV-related tumors are caused by the HPV16 type.

For cervical intraepithelial neoplasia (CIN), which is a precancerous lesion of cervical cancer, the only treatment in the related art has been a conization or laser vaporization, and there exists no drug therapy.

The present inventors have developed a pharmaceutical composition for treating HPV infection and HPV-related tumor (see, for example, Patent Document 1). The pharmaceutical composition has been subjected to a phase I/II clinical trial and is in the clinical trial stage.

### Citation List

### Patent Document

Patent Document 1: Japanese Patent No. 6464201

### SUMMARY OF INVENTION

### Technical Problem

All of the therapeutic drugs for HPV infection and HPV-related tumor, which have been developed so far, are immunotherapeutic agents that induce cellular immunity against HPVs. However, the correlation between an ability to induce anti-HPV-specific immunity and a clinical therapeutic effect is unclear, and the therapeutic effect on CIN is also limited. It is presumed that a gap between these therapeutic drugs and the systemic immunity is created from the viewpoint that the systemic immunity is induced by intramuscular injection, whereas the local environment of the cervix with the CIN lesion is the environment of mucosal immunity. Under such circumstances, there is still no companion diagnostic for a therapeutic drug for HPV infection and HPV-related tumor.

Furthermore, it has been clarified that the pharmaceutical composition described in Patent Document 1, which has been developed by the present inventors, is efficacious for about 40% of patients with CIN, but is not efficacious for the remaining patients with CIN, as shown in Examples which will be described later. Therefore, there is a demand for a companion diagnostic for the pharmaceutical composition.

The present invention has been made in consideration of the circumstances, and provides a novel method and a novel kit for predicting the efficacy of a treatment with immunotherapeutic agent on a patient with mucosal lesion.

### Solution to Problem

That is, the present invention includes the following aspects.
(1) A method for predicting efficacy of a treatment with immunotherapeutic agent on a patient with mucosal lesion, the method including:
   a step of measuring an expression level of CD86 in a biological sample derived from the patient with mucosal lesion.
(2) The method according to (1), further including:
   a step of evaluating or predicting that the treatment with immunotherapeutic agent is potentially efficacious in a case where the expression level of CD86 is equal to or less than a preset reference value.
(3) The method according to (1) or (2),
   in which the mucosal lesion is human papillomavirus infection or human papillomavirus-related tumor.
(4) The method according to (3),
   in which the human papillomavirus-related tumor is cervical intraepithelial neoplasia.
(5) The method according to any one of (1) to (4),
   in which the biological sample is exfoliated cervical cells.
(6) The method according to any one of (1) to (5),
   in which the immunotherapeutic agent is a drug that induces anti-human papillomavirus cellular immunity.
(7) A kit for use in prediction of efficacy of a treatment with immunotherapeutic agent on a patient with mucosal lesion, the kit including:
   a substance that specifically binds to CD86, or a forward primer and a reverse primer for amplifying a nucleic acid that encodes CD86.
(8) A kit for use in the method according to any one of (1) to (6), the kit including:
   a substance that specifically binds to CD86, or a forward primer and a reverse primer for amplifying a nucleic acid that encodes CD86.
(9) The kit according to (8),
   in which the substance that specifically binds to CD86 is anti-CD86 antibody.

### Advantageous Effects of Invention

By the method and the kit of the aspect, the efficacy of a treatment with immunotherapeutic agent on a patient with mucosal lesion can be predicted.

### BRIEF DESCRIPTION OF DRAWINGS

[FIG. 1] A graph showing the expression level of each factor in CR/PR cases and SD cases by an RT-PCR method in Example 1.
[FIG. 2] A graph showing a CR/PR rate in Example 1.
[FIG. 3] A graph showing the expression level of each factor in CR cases and non-CR cases by an RT-PCR method in Example 2.
[FIG. 4] A graph showing CR rates in Example 2.
[FIG. 5] A graph showing a partial correlation matrix between the expression level of CD86 and other factors correlated therewith in Example 2.

### DESCRIPTION OF EMBODIMENTS

### [Definitions]

The term "includes" (comprises) means that constituent elements other than the target constituent elements may be included. The term "consists of" means that constituent elements other than the target constituent elements are not included. The term "consists essentially of" means that constituent elements other than the target constituent elements are not included in a manner whereby a special function is exhibited (such as a manner in which the effect of the invention is completely lost). In the present specification, the term "includes" (comprises) encompasses the meanings of "consists of" and "consists essentially of".

Proteins, peptides, nucleic acids, and cells can be isolated. "Isolated" means in a natural state or a state separated from other components. "Isolated" may mean substantially not including other components. "Substantially not including other components" means that the amount of other components included in the isolated components is negligible. The amount of other components included in the isolated component may be, for example, 10% by mass or less, 5% by mass or less, 4% by mass or less, 3% by mass or less, 2% by mass or less, 1% by mass or less, 0.5% by mass or less, or 0.1% by mass or less. The proteins, peptides, nucleic acids, and cells described in the present specification can be isolated proteins, isolated peptides, isolated nucleic acids, and isolated cells.

Hereinafter, the method for predicting the efficacy of a treatment with immunotherapeutic agent for a patient with mucosal lesion, and the kit for use in the method according to one embodiment of the present invention will be described in detail.

### <Mucosal Lesion>

In the present specification, the "mucosal lesion" refers to a disease in which a neoplastic lesion or an infectious lesion is formed on the mucosa due to inflammation or infection. Specific examples of the mucosal lesion include, but are not limited to, neoplastic lesions caused by infections withhuman papillomavirus (HPV), Epstein-Barr (EB) virus, Helicobacter pylori, and the like; and infectious lesions that cause lesions on the genital mucosa, such as genital chlamydia, genital herpes, gonorrhea, and genital warts.

Among these, the method and the kit of the present embodiment are preferably applied to human papillomavirus infection or human papillomavirus-related tumor.

Examples of the HPV-related tumor include, but are not limited to, oral epithelial dysplasia, laryngeal squamous epithelial dysplasia, cervical intraepithelial neoplasia, anal intraepithelial neoplasia, vaginal intraepithelial neoplasia, vulvar intraepithelial neoplasia, and penile intraepithelial neoplasia. Among these, the method and the kit of the present embodiment are particularly preferably applied to cervical intraepithelial neoplasia.

### <Immunotherapeutic Agent for Patient with Mucosal Lesion>

The immunotherapeutic agent for a patient with mucosal lesion refers to a drug that treats mucosal lesion by using or modifying an immune system. More specifically, the immunotherapeutic agent is a drug having an action of improving an immune response to mucosal lesion cell and/or a causative pathogen. The immunotherapeutic agent may be a known drug or a new drug. The immunotherapeutic agent may improve humoral immunity against mucosal lesion cell and/or a causative pathogen, or may improve cellular immunity against mucosal lesion cell and/or a causative pathogen.

The immunotherapeutic agent includes immune checkpoint inhibitor.

In the present specification, "immune checkpoint (system)" refers to a mechanism that suppresses immune responses by controlling the activity of immune-related cells (including T cells). This system suppresses inappropriate immune responses and excessive inflammatory responses to the cells and tissues of the self. Immune checkpoint molecules are molecules that are expressed on immune-related cells and that transmit signals that suppress immune responses to such immune-related cells by binding to ligands.

Examples of immune checkpoint molecules and ligands thereof include, but are not limited to, molecules such as PD-1, CTLA-4, TIM-3 (T cell immunoglobulin and mucin containing protein-3), LAG-3 (lymphocyte activation gene-3), PD-L1, PD-L2, BTNL2, B7-H3, B7-H4, B7-H5 (VISTA), CD48, CD80, 2B4, BTLA, CD160, CD60, CD86, TIGIT (T cell Immunoreceptor with Ig and ITIM domain).

In the present specification, "immune checkpoint inhibitor" refers to a drug that inhibits signal transmission by immune checkpoint molecules by inhibiting the binding of immune checkpoint molecules to the ligands thereof.

Examples of the known immune checkpoint inhibitors include PD-1 inhibitor, PD-L1 inhibitor, and CTLA-4 inhibitor.

Examples of the PD-1 inhibitor include, but are not limited to, anti-PD-1 antibodies such as Nivolumab (marketed as Opdivo (registered trademark)) and Pembrolizumab (marketed as Keytruda (registered trademark)).

Examples of the PD-L1 inhibitor include, but are not limited to, anti-PD-L1 antibodies such as Avelumab, Durvalumab, and Atezolizumab (marketed as Tecentriq (registered trademark)).

Examples of the CTLA-4 inhibitor include, but are not limited to, Anti-CTLA-4 antibodies such as Ipilimumab and Tremelimumab.

Examples of other immune checkpoint inhibitors include drugs that target immune checkpoint proteins such as TIM-3, LAG-3, B7-H3, B7-H4, B7-H5 (VISTA), and TIGIT.

In a case where the mucosal lesion is HPV infection or HPV-related tumor, a drug that induces anti-HPV cellular immunity is preferably used as a known immunotherapeutic agent.

As the drug that induces anti-HPV cellular immunity, for example, the therapeutic drug containing a polypeptide derived from E7 protein of HPV, which is described in Japanese Patent No. 6464201, can also be used. The polypeptide derived from the E7 protein of the HPV may be a full-length protein of the E7 protein of the HPV, or may be a protein in which one or several amino acids are deleted, substituted, or added. The polypeptide derived from the E7 protein of the HPV may be derived from wild-type E7 protein or may be derived from mutant E7 protein.

Among the polypeptides derived from the E7 protein of the HPV, mutated E7 protein in which amino acid residues involved in the binding of the E7 protein to the Rb protein correspond to Asp at position 21, Cys at position 24, and Glu at position 26 in the wild-type E7 protein (SEQ ID NO: 1) of HPV16 is preferable, and a mutated E7 protein (SEQ ID NO: 2) in which all of Asp at position 21, Cys at position 24, and Glu at position 26 in the wild-type E7 protein (SEQ ID NO: 1) of HPV16 are substituted with Gly is more preferable.

As the drug that induces anti-HPV cellular immunity, a bacterium having a polypeptide derived from the E7 protein of the HPV on the bacterial cell surface may be used. The bacterium is preferably a lactic acid bacterium, more preferably a lactic acid bacterium belonging to the Lactobacillus genus, and still more preferably Lactobacillus casei. The lactic acid bacterium may be used alone or in combination of two or more kinds thereof.

The polypeptide derived from the E7 protein of the HPV is preferably bound to the bacterial cell surface of the bacterium. Examples of a method for binding the polypeptide derived from the E7 protein of the HPV to the bacterial cell surface include a method for forming a fusion protein with an anchor protein. Examples of the anchor protein include a cA anchor protein, which is a cell wall-binding protein derived from AcmA, which is a peptidoglycan hydrolase of Lactococcus lactis, and a prtP anchor protein of prtP, which is a protease of Lactobacillus casei. More specifically, the polypeptide derived from the E7 protein of the HPV may be a fusion protein with a cA anchor protein derived from AcmA, or a protein expressed from a sequence in which a nucleic acid encoding the polypeptide derived from the E7 protein of the HPV is bound to a PrtP anchor gene.

The amount of the polypeptide derived from the E7 protein of the HPV on the bacterial cell surface is preferably 0.03 µg to 1.0 µg per 1 × 10⁸ bacterial cells of the lactic acid bacterium. For example, as the drug that induces anti-HPV cellular immunity, the lactic acid bacterium-containing composition described in Japanese Patent No. 6464201 can also be used.

### <CD86>

CD86 is a major molecule that transmits a co-stimulatory signal to antigen-specific T cells, and is also classified as a B7 family molecule and also referred to as a B7.2 molecule. CD86 is expressed on antigen-presenting cells (APCs) such as monocytes and dendritic cells, and binds to CD28 on T cells. In a case where CD86 binds to CD28, proliferation of T cells, production of cytokines, promotion of cell survival, and the like occur due to a signal from CD28, and thus acts on the activation of T cells (accelerating action). On the other hand, a cytotoxic T-lymphocyte antigen 4 (CTLA4) is expressed on activated T cells or regulatory T cells, binds to CD86 more strongly than to CD28, and thus, controls the activation of T cells (braking action).

As shown in Examples which will be described later, the present inventors have analyzed the expression level of CD86 in exfoliated cervical cells derived from patients with moderate dysplasia (CIN2) and patients with severe dysplasia/carcinoma in situ (CIN3) in cervical intraepithelial neoplasia, in which the present inventors have clarified the susceptibility to a pharmaceutical composition for treating at least any one of an HPV infection or an HPV-related tumor developed by the inventors (see Japanese Patent No. 6464201). As a result, CD86 was found as a factor in which the value of the area under the curve (AUC) of the ROC curve serving as an index of the discrimination ability in the drug sensitivity determination was 0.75 or more.

Examples of the amino acid sequence of human CD86 include Genbank accession numbers NP_001193853.2, NP_001193854.2, NP_008820.4, NP_787058.5, and NP_795711.2.

Examples of the base sequences of human CD86 include GenBank accession numbers NM_001206924.2, NM_001206925.2, NM 006889.5, NM_175862.5, and NM_176892.2.

### <Method for Predicting Efficacy of Treatment with Immunotherapeutic Agent for Patient with Mucosal Lesion>

The method for predicting the efficacy of a treatment with immunotherapeutic agent for a patient with mucosal lesion according to one embodiment of the present invention (which may hereinafter be simply referred to as "the method of the present embodiment") is a method including a step of measuring an expression level of CD86 in a biological sample derived from the patient with mucosal lesion (which may hereinafter be referred to as "a measurement step"). The measurement step according to the present aspect is a step performed in vitro.

By the method of the present embodiment, the efficacy of a treatment with immunotherapeutic agent on a patient with mucosal lesion can be predicted. That is, the method of the present embodiment can also be referred to as a companion diagnostic method forimmunotherapeutic agent for mucosal lesion.

In the present specification and the claims, the term "companion diagnosis" refers to an examination performed before the actual initiation of medication to predict the effect of a drug for individual patients with mucosal lesion, the risk of side effects, and the appropriate dosage of the medication. It is preferable that the "companion diagnosis" is an examination performed before the start of administration of the immunotherapeutic agent in order to predict the efficacy of the treatment with the immunotherapeutic agent for a patient with mucosal lesion.

Hereinafter, the method of the present embodiment will be described in detail.

Examples of the biological sample used in the method of the present embodiment include cells of a lesion part or a homogenate thereof. The biological sample may include immune cells (T cells and the like) present in the lesion part in addition to the cells in the lesion part. In a case where the mucosal lesion to which the method of the present embodiment is applied is cervical intraepithelial neoplasia, it is preferable to use exfoliated cervical cells as the biological sample. The exfoliated cervical cells are cells obtained by scraping the cervix with a scraping instrument. The exfoliated cervical cells can be collected using, for example, a commercially available collection instrument used for cervical cancer screening, such as liquid-based cytology ThinPrep (registered trademark) manufactured by Hologic Japan, Inc. The exfoliated cervical cells may include immune cells present in the cervix in addition to the cervical cells.

The species from which the biological sample is derived is not particularly limited, and examples thereof include mammals such ashuman, monkey, marmoset, dog,cat, cow, horse, sheep, pig, rabbit, mouse, rat, guinea pig, and hamster. Among these, the human-derived sample is preferable.

Biological samples may be pre-treated, such as by being added to a known buffer, and it is also possible to use samples collected from a living organism as they are.

In the measurement step, the method for measuring the expression level of CD86 includes at least one of the following (1) and (2).
(1) The expression level of the CD86 protein is measured.
(2) The amount of the nucleic acid fragment having a nucleic acid or a partial sequence thereof, which encodes the CD86 protein, is measured.

Examples of the method of (1) include immunological methods such as a Western blotting method using a substance that specifically binds to CD86, an enzyme-linked immunosorbent assay (ELISA) method, a chemiluminescent enzyme immuno assay (CLEIA) method, and an immunoprecipitation method.

The Western blotting method is, for example, a method in which a sample containing CD86 is subjected to acrylamide gel electrophoresis to separate CD86, which is transferred to a membrane and reacted with an antibody (primary antibody) capable of recognizing CD86, the primary antibody that is not bound to CD86 is removed by washing or the like, and then the generated immune complex of CD86 and the primary antibody is detected using a labeled secondary antibody. The amount of CD86 present in the biological sample can be measured by measuring the amount of the label of the labeled secondary antibody bound to the generated immune complex.

The immunohistochemical staining method is a method in which a tissue section, a cell, or the like fixed on a slide glass is reacted with an antibody to generate an immune complex, a primary antibody that is not bound to CD86 is removed by washing or the like, and then the immune complex is detected by a labeled secondary antibody bound to the immune complex, and is efficacious for analyzing an expression site of CD86 in a tissue or a cell.

A substance that specifically binds to CD86, such as a specific antibody to CD86 to be used (which may hereinafter be referred to as a "CD86-specific antibody"), can be used as exemplified in the kit described later.

Examples of the method of (2) include, but are not limited to, a quantitative RT-PCR method, a Northern blot method, and an in situ hybridization method. The nucleic acid fragment having a nucleic acid measured by the method of (2), or a partial sequence thereof is a nucleic acid fragment having an mRNA encoding a CD86 protein, or a partial sequence thereof.

Such a method can use a probe or a primer created based on the known base sequence information of CD86, or a combination thereof.

As the method of (2), a LAMP method can also be used. The LAMP method is an isothermal gene amplification technique developed by Eiken Chemical Co., Ltd., which defines 6 regions of the target gene (F3c, F2c, and F1c from the 3' end side, and B3, B2, and B1 from the 5' end side), and amplifies the regions using 4 types of primers (an FIP primer, an F3 primer, a BIP primer, and a B3 primer) for the 6 regions.

In the method of the present embodiment, the efficacy of the treatment with immunotherapeutic agent in a patient with mucosal lesion, from which the biological sample to be evaluated is derived, is predicted on the basis of the measured expression level of CD86.

Specifically, in a case where the expression level of CD86 is equal to or less than a preset reference value, it can be evaluated or predicted that the patient with mucosal lesion, from which the biological sample to be evaluated is derived, is potentially sensitive to immunotherapeutic agent, that is, the treatment with the immunotherapeutic agent is potentially efficacious. On the other hand, in a case where the expression level of CD86 is more than a preset reference value, it can be evaluated or predicted that the patient with mucosal lesion, from which the biological sample to be evaluated is derived, is not potentially sensitive to the immunotherapeutic agent, that is, the treatment with the immunotherapeutic agent is not potentially efficacious. Therefore, the method of the present embodiment may further include, in addition to the measurement step, a step of evaluating or predicting that the treatment with the immunotherapeutic agent may be efficacious in a case where the expression level of CD86 is equal to or less than a preset reference value (hereinafter also referred to as an "evaluation step").

In a case where the mucosal lesion is HPV infection or HPV-related tumor, the administered immunotherapeutic agent is a drug that induces anti-HPV cellular immunity, and the expression level of CD86 is more than a preset reference value, it is shown that the activity of CTLA-4-positive Foxp3+ Treg in a lesion site is increased in a patient with HPV infection or a patient with HPV-related tumor, from which the biological sample to be evaluated is derived. Therefore, it is considered that the anti-HPV cellular immunity induced by the immunotherapeutic agent is suppressed at the lesion site. Accordingly, it is possible to evaluate or predict the potential effect of a use of the drug that induces anti-HPV cellular immunity in combination with the CTLA-4 inhibitor that is an immune checkpoint inhibitor on HPV infection or HPV-related tumor.

The above-described reference value is a reference value for identifying a patient group with mucosal lesion having sensitivity to an immunotherapeutic agent (responder group) and a patient group with mucosal lesion having no sensitivity to an immunotherapeutic agent (non-responder group).

The reference value can be experimentally determined, for example, as a threshold value that can distinguish between the responder group and the non-responder group by measuring the expression level of CD86 present in the biological samples of the two groups. A method for determining the reference value of the expression level of CD86 in the method of the present embodiment is not particularly limited, and for example, the reference value can be determined using a general statistical method.

Specifically, as the method for determining the reference value, for example, the expression level of CD86 in a biological sample collected in advance before the administration of an immunotherapeutic agent is measured for a patient diagnosed as having mucosal lesion by a method such as image diagnosis, cytodiagnosis, and histodiagnosis. After measuring the expression level of CD86 for a plurality of patients, the average value or median value, and the like thereof are calculated by a statistical method, and these values can be used as the reference value. In addition, it is also preferable that the reference value is described in the instructions or the like of the kit relating to the evaluation or prediction method.

In addition, the expression level of CD86 present in the biological sample collected in advance before the administration of the immunotherapeutic agent is measured for each of a plurality of responder groups and a plurality of non-responder groups, and a threshold value is determined such that the responder group and the non-responder group are distinguished from each other by ROC analysis or the like, and used as the reference value.

### <Other Steps>

The method of the present embodiment may include other steps in addition to the steps. Examples of the other steps include (a) a step of administering an immunotherapeutic agent to a patient with a mucosal lesion, based on evaluation results of an expression level of CD86 in a case where the expression level of CD86 is equal to or less than a preset reference value, and (b) a step of administering a drug that induces anti-HPV cellular immunity in combination with a CTLA-4 inhibitor that is an immune checkpoint inhibitor to a patient with a mucosal lesion in a case where the mucosal lesion is an HPV infection or an HPV-related tumor, the administered immunotherapeutic agent is the drug that induces anti-HPV cellular immunity, and the expression level of CD86 is more than a preset reference value. In a case of including any of the step (a) or (b), the method of the present embodiment may be a method for treating a mucosal lesion.

### (Step (a))

In a case where an expression level of CD86 is equal to or less than a preset reference value, a treatment policy for a mucosal lesion can be determined based on the expression level, and for example, an immunotherapeutic agent may be administered to a patient with a mucosal lesion. In a case where the expression level of CD86 is equal to or less than a preset reference value, it is highly likely that the patient with a mucosal lesion from which the biological sample to be evaluated is derived has sensitivity to an immunotherapeutic agent, and it is predicted that the treatment with the immunotherapeutic agent is efficacious. Therefore, the immunotherapeutic agent can be administered to a patient with a mucosal lesion in which the expression level of CD86 is equal to or less than a preset reference value.

On the other hand, it is predicted that a patient with mucosal lesion in which the expression level of CD86 is more than a preset reference value has low sensitivity to immunotherapeutic agent. Therefore, for the patient with mucosal lesion in which the expression level of CD86 is more than a preset reference value, a careful follow-up of symptoms and a treatment of mucosal lesions may be performed. Examples of the treatment method include a treatment method other than the administration of immunotherapeutic agent. Depending on the type and the symptoms of the mucosal lesion, for example, surgical therapy, chemotherapy, radiotherapy, or the like may be performed. For a patient with mucosal lesion in which the expression level of CD86 is more than a preset reference value, it is predicted that the immune response to the mucosal lesion is in a suppressed state, and thus an immunotherapeutic agent may be administered together with an immune checkpoint inhibitor.

### (Step (b))

In a case where the mucosal lesion is an HPV infection or an HPV-related tumor, the administered immunotherapeutic agent is a drug that induces anti-HPV cellular immunity, and the expression level of CD86 is more than a preset reference value, an immune checkpoint inhibitor may be administered.

In a case where the expression level of CD86 is more than a preset reference value, it is predicted that the patient with HPV infection or a patient with HPV-related tumor has a low susceptibility to a drug that induces anti-HPV cellular immunity. Such a patient is predicted to have a suppressed immune response to HPV infection or HPV-related tumor, and thus the administration of an immune checkpoint inhibitor may be performed to reduce the immune suppression by an immune checkpoint system. Therefore, for the patient with HPV-infection or the patient with HPV-related tumor in which the expression level of CD86 is more than a preset reference value, the treatment of the HPV infection or the HPV-related tumor may be performed according to the type and the symptoms of the disease. In addition to the administration of the immune checkpoint inhibitor, surgical therapy, chemotherapy, radiotherapy, or the like may be performed as the treatment method.

### <Kit>

The kit of the present embodiment is used for predicting the efficacy of a treatment with immunotherapeutic agent on a patient with mucosal lesion. The kit of the present embodiment is preferably a kit for use in the above-described method. The kit of the present embodiment includes a substance that specifically binds to CD86, or a forward primer and a reverse primer for amplifying a nucleic acid that encodes CD86.

By measuring an expression level of CD86 in a biological sample derived from a patient with mucosal lesion, using the kit of the present embodiment, it is possible to predict the efficacy of the treatment with immunotherapeutic agent for the patient with mucosal lesion. That is, the kit of the present embodiment can also be referred to as a companion diagnostic or companion diagnostic kit for immunotherapeutic agent for mucosal lesion.

### [Specific Binding Substance]

The substance specifically binding to CD86, which is included in the kit of the present embodiment, may be of one kind or of two or more kinds, and may be any of substances that make it possible to measure the expression level of CD86.

Examples of the substance that specifically binds to CD86 include anti-CD86 antibody and aptamer that binds to CD86. Among these, the anti-CD86 antibody is preferable.

The anti-CD86 antibody may be a polyclonal antibody, a monoclonal antibody, or a functional fragment of the antibody. Among these, the CD86-specific antibody is preferably the monoclonal antibody since it has high specificity and excellent quantitativeness.

The anti-CD86 antibody may be a commercially available product or may be manufactured by a method which will be described later.

In the present specification, "specifically binding" means that an antibody binds only to a target protein (antigen), and the binding can be quantified, for example, by the binding of the antibody to an epitope of an antigen in an assay in a test tube, and preferably a plasmon resonance assay using a purified wild-type antigen (for example, BIAcore, GE-Healthcare Uppsala, and Sweden). The affinity of binding can be defined by ka (a rate constant related to the antibody binding from an antigen-antibody binding complex), kD (a dissociation constant), and KD (kD/ka). The binding affinity (KD) in a case where the antibody specifically binds to the antigen is preferably 10⁻⁸ mol/L or less, and more preferably 10⁻¹³ mol/L or more and 10⁻⁹ mol/L or less.

In the present embodiment, the term "polyclonal antibody" means an antibody preparation including different antibodies against different epitopes. That is, in a case where the antibody of the present embodiment is a polyclonal antibody, it may include different antibodies that specifically bind to CD86.

The term "monoclonal antibody" means an antibody (including an antibody fragment) obtained from a population of substantially homogeneous antibodies.

In contrast to the polyclonal antibody, the monoclonal antibody means an antibody that recognizes a single determinant on an antigen.

In the present embodiment, the "functional fragment" of an antibody means a part (partial fragment) of the antibody, which specifically recognizes a target protein. Specific examples thereof include Fab, Fab', F(ab')2, a variable region fragment (Fv), a disulfide-bonded Fv, a single-chain Fv (scFv), sc(Fv)2, a diabody, a multispecific antibody, and polymers thereof.

An aptamer is a substance having a specific binding ability to a target substance. Examples of the aptamer include a nucleic acid aptamer and a peptide aptamer. It is possible to select nucleic acid aptamers having a specific binding ability to antigens, for example, by a systematic evolution of ligands by exponential enrichment (SELEX) method. In addition, a peptide aptamer having an ability to specifically bind to an antigen can be screened by, for example, a Two-hybrid method using yeast.

A labeling substance or a modifying substance may be bound to the substance that specifically binds to CD86.

Examples of the labeling substance include a stable isotope, a radioactive isotope, a fluorescent substance, an enzyme, and a magnetic substance. Among these, the fluorescent substance or the enzyme is preferable as the labeling substance from the viewpoint of easy detection and high sensitivity. In a case where the substance that specifically binds to CD86 includes the labeling substance, CD86 can be detected and quantified simply and with high sensitivity.

Examples of the stable isotope include, but are not limited to, ¹³C, ¹⁵N, ²H, ¹⁷O, and ¹⁸O.

Examples of the radioactive isotope include, but are not limited to, ³H, ¹⁴C, ¹³N, ³²P, ³³P, and ³⁵S.

Examples of the fluorescent substance include, but are not limited to, a cyanine dye (for example, Cy3 and Cy5), a rhodamine 6G reagent, and other known fluorescent dyes (for example, GFP, fluorescein isothiocyanate (FITC), and TAMRA).

Examples of the enzyme include an alkaline phosphatase and a peroxidase (HRP). In a case where the labeling substance is the enzyme, it is preferable to use an enzyme substrate. As the enzyme substrate, in a case of the alkaline phosphatase, p-nitropheny phosphase (pNPP), 4-methylumbelliferyl phosphate (4-MUP), or the like can be used, and in a case of the enzyme peroxidase, 3,3'-diaminobenzidine (DAB), 3,3',5,5'-tetramethylbenzidine (TMB), o-phenylenediamine (OPD), 2,2-azino-di-(3-ethylbenzothiazoline-6-sulfonic acid) (ABTS), 10-acetyl-3,7-dihydroxyphenoxazine (ADHP), or the like can be used.

Examples of the magnetic substance include, but are not limited to, gadolinium, Gd-DTPA, Gd-DTPA-BMA, Gd-HP-DO3A, iodine, iron, iron oxide, chromium, manganese, or a complex or chelate complex thereof.

Examples of the modifying substance include biotin and an Fc fragment. In a case where the substance that specifically binds to CD86 includes a modifying substance, CD86 can be detected and quantified simply and with high sensitivity using a specific binding substance (for example, a specific antibody; or avidin or streptavidin in a case of a specific binding substance to biotin) to the modifying substance to which the labeling substance is bound.

### (Method for Manufacturing Antibody)

In a case where the anti-CD86 antibody is a polyclonal antibody, it can be obtained by immunizing an animal to be immunized with an antigen (for example, ephrin B such as syntaxin or a fragment thereof, or cells that express these), and purifying the antiserum by a means in the related art (for example, salting out, centrifugation, dialysis, and column chromatography).

CD86 used as an antigen, or a peptide fragment having a part of an amino acid sequence thereof may be chemically synthesized or may be manufactured by a known genetic engineering method based on known base sequence information of CD86.

In a case where the CD86-specific antibody is a monoclonal antibody, it can be manufactured by a hybridoma method or a recombinant DNA method.

Examples of the hybridoma method include a Kohler and Milstein method (see, for example, Kohler & Milstein, Nature, 256: 495 (1975)). Examples of the antibody-producing cell used in the cell fusion step of this method include spleen cells, lymph node cells, and peripheral blood leukocytes of animals (for example, mice, rats, hamsters, rabbits, monkeys, and goats) immunized with an antigen (CD86 or a fragment thereof, a cell expressing CD86 or a fragment thereof, and the like). In addition, antibody-producing cells obtained by allowing an antigen to act on the above-described cells, lymphocytes, or the like, which have been isolated in advance from a non-immunized animal, in a culture medium can also be used. As the myeloma cell, various known cell lines can be used. The antibody-producing cell and the myeloma cell may be derived from different animal species as long as they are capable of being fused with each other, but are preferably derived from the same animal species. Examples of a method for obtaining a hybridoma include a method for obtaining a hybridoma which is produced by cell fusion between a spleen cell obtained from a mouse immunized with an antigen and a mouse myeloma cell, and produces a monoclonal antibody specific for a target protein by subsequent screening. Examples of a method for obtaining a monoclonal antibody produced by a hybridoma include, for a monoclonal antibody against a target protein, a method for obtaining the monoclonal antibody by culturing the hybridoma, or a method for obtaining the monoclonal antibody from ascites of a mammal to which the hybridoma has been administered.

Examples of the recombinant DNA method include a method in which DNA encoding the antibody or a functional fragment of the antibody is cloned from a hybridoma, a B cell, or the like, incorporated into an appropriate vector, and transferred to a host cell (for example, a mammalian cell line, Escherichia coli, a yeast cell, an insect cell, and a plant cell) to produce the antibody of the present embodiment as a recombinant antibody (see, for example, P. J. Delves, Antibody Production: Essential Techniques, 1997 WILEY, P. Shepherd and C. Dean Monoclonal Antibodies, 2000 OXFORD UNIVERSITY PRESS, Vandamme A. M. et al., Eur. J. Biochem. 192: 767-775 (1990)).

In the expression of a DNA encoding an antibody, a host cell may be transformed by separately incorporating a DNA encoding a heavy chain or a light chain into an expression vector, or a host cell may be transformed by incorporating a DNA encoding a heavy chain and a light chain into a single expression vector (see, for example, PCT International Publication No. WO94/11523). The antibody can be acquired in a substantially pure and uniform form by culturing the host cell, and separating and purifying the host cell within the host cell or from the culture solution. For the separation and the purification of the antibody, a method generally used for the purification of a polypeptide can be used. Examples of a method using transgenic animal manufacturing technology include a method in which a transgenic animal (for example, a cow, a goat, a sheep, or a pig) into which an antibody gene has been incorporated is manufactured, and a large amount of a monoclonal antibody derived from the antibody gene from the milk of the transgenic animal is acquired.

As long as the antibody can specifically bind to CD86, it may also be an amino acid sequence variant.

The amino acid sequence variant can be manufactured by introducing a mutation into a DNA encoding an antibody chain or by peptide synthesis. The site where the amino acid sequence of the antibody is modified may be a constant region of a heavy chain or a light chain of the antibody, or may be a variable region (a framework region and a CDR) as long as the antibody has the same activity as the antibody before the modification. In addition, a method for screening an antibody having an increased affinity for an antigen by modifying an amino acid of a CDR may be used (see, for example, PNAS, 102: 8466-8471 (2005), Protein Engineering, Design & Selection, 21: 485-493 (2008), PCT International Publication No. WO2002/051870, J. Biol. Chem., 280: 24880-24887 (2005), Protein Engineering, Design & Selection, 21: 345-351 (2008)).

With regard to the number of amino acids to be modified, the number of the amino acids is preferably 10 or less, more preferably 5 or less, and most preferably 3 or less (for example, 2 or less, and 1). The modification of the amino acids is preferably a conservative substitution.

In the present specification, the "conservative substitution" means substitution with another amino acid residue having a chemically similar side chain. A group of amino acid residues having chemically similar amino acid side chains is well known in the technical field to which the present invention belongs. For example, the amino acids can be classified into acidic amino acids (aspartic acid and glutamic acid), basic amino acids (lysine, arginine, and histidine), and neutral amino acids, such as amino acids having a hydrocarbon chain (glycine, alanine, valine, leucine, isoleucine, and proline), amino acids having a hydroxy group (serine and threonine), amino acids including sulfur (cysteine and methionine), amino acids having an amide group (asparagine and glutamine), amino acids having an imino group (proline), amino acids having an aromatic group (phenylalanine, tyrosine, and tryptophan), and the like. It is preferable that the amino acid sequence variant has a higher antigen-binding activity than a control antibody.

In the present embodiment, the antigen-binding activity of the antibody (including the functional fragment, the amino acid sequence variant, and the like of the antibody described above) can be evaluated by, for example, an ELISA method, a Western blotting method, an immunoprecipitation method, or an immunostaining method.

### (Labeling Reagent)

The kit of the present embodiment may further include, as a labeling reagent, a reagent in which a labeling substance is bound to a substance that specifically binds to CD86 (for example, a reagent in which a labeling substance is bound to an anti-CD86 antibody), or a reagent in which a labeling substance is bound to an antibody against a substance that specifically binds to CD86 (for example, a reagent in which a labeling substance is bound to an antibody against an anti-CD86 antibody), in addition to the substance that specifically binds to CD86.

For example, in a case where the reagent in which a labeling substance is bound to anti-CD86 antibody is provided as a labeling reagent, it is possible to detect and quantify CD86 by using a sandwich enzyme-linked immunosorbent assay (ELISA) method, a chemiluminescent enzyme immunoassay method (CLEIA method), or the like based on an antigen measurement system.

For example, in a case where a reagent in which a labeling substance is bound to an antibody against anti-CD86 antibody is used as the labeling reagent, CD86 can be detected and quantified by using an ELISA method using an antibody measurement system, an indirect fluorescence antibody method, a CLEIA method using an antibody measurement system, or the like.

The antibody against the anti-CD86 antibody is preferably an antibody against an animal (for example, mouse, rat, hamster, rabbit, monkey, and goat) from which the anti-CD86 antibody is derived. For example, in a case where the anti-CD86 antibody is derived from mouse, it is preferably an anti-mouse antibody. In addition, all classes and subclasses of immunoglobulins are included in the reagent in which a labeling substance is bound to an antibody against the anti-CD86 antibody.

The kit of the present embodiment may further include a reaction stopping solution, as necessary. Examples of the reaction stopping solution include sulfuric acid and sodium hydroxide.

### [Primer Set]

Hereinafter, a forward primer and a reverse primer for amplifying a nucleic acid that encodes CD86 may be referred to as a "primer set".

The primer set can be used in a case of measuring the expression level of a nucleic acid that encodes CD86, that is, the expression level of an mRNA, by an RT-PCR method or the like.

The forward primer can have a sequence specific to the base sequence of the 3' end region of a nucleic acid that encodes CD86.

The reverse primer can have a sequence specific to the base sequence of the 5' end region of a nucleic acid that encodes CD86.

In a case where the forward primer and the reverse primer have the sequences, the nucleic acid that encodes CD86 can be more specifically amplified in a real-time PCR reaction.

The forward primer and the reverse primer for amplifying a nucleic acid that encodes CD86 can be appropriately designed by those skilled in the art, based on the base sequence information of CD86. Alternatively, commercially available forward and reverse primers for amplifying a nucleic acid that encodes CD86 may be used. For example, Assay ID No. Hs01567026_m1 manufactured by Thermo Fisher Scientific Inc. can be used.

The kit of the present embodiment can further include a random primer for a reverse transcription reaction, in addition to the above-described primer set.

Examples of the random primer for the reverse transcription reaction include a mixture of 6-mer or 9-mer deoxyribonucleotides having a random sequence, and the 5' end of the deoxyribonucleotide is preferably phosphorylated.

The kit of the present embodiment may further include a labeled oligonucleotide probe. The labeled oligonucleotide probe includes a base sequence complementary to at least three bases of a base sequence of a nucleic acid that encodes CD86. The probe can specifically hybridize with an amplification product from the forward primer and the reverse primer by including the base sequence.

As the labeled oligonucleotide probe, for example, an oligonucleotide probe in which a quencher is bound to the 5' end and a labeling substance is bound to the 3' end can be used. By providing the quencher at the 5' end and the labeling substance at the 3' end, in a case of an extension reaction by a DNA polymerase in a real-time PCR reaction, a probe that has hybridized to a nucleic acid that encodes CD86 is decomposed by the 5'-3' exonuclease activity of the polymerase, and the suppression by the quencher is released, making it possible to detect the labeling substance.

Examples of the labeling substance bound to the 3' end of the probe include a fluorescent dye, a fluorescent bead, a quantum dot, biotin, an antibody, an antigen, an energy absorbing substance, a radioisotope, a chemiluminescent substance, and an enzyme.

Examples of the fluorescent dye include carboxyfluorescein (FAM), 6-carboxy-4',5'-dichloro-2',7'-dimethoxyfluorescein (JOE), fluorescein isothiocyanate (FITC), tetrachlorofluorescein (TET), 5'-hexachloro-fluorescein-CE phosphoramidite (HEX), Cy3, Cy5, Alexa 568, and Alexa 647.

In a case where the labeling substance bound to the 3' end of the probe is a fluorescent dye, the combination of the labeling substance bound to the 3' end of the probe and the quencher bound to the 5' end of the probe is preferably a combination of labeling substances that can cause fluorescence (foerster) resonance energy transfer (FRET). Specific examples of the combination include a combination of a fluorescent dye having an excitation wavelength of approximately 490 nm (for example, FITC, rhodamine green, Alexa (registered trademark) fluor 488, and BODIPY FL) and a fluorescent dye having an excitation wavelength of approximately 540 nm (for example, TAMRA, tetramethylrhodamine, and Cy3), or a combination of a fluorescent dye having an excitation wavelength of approximately 540 nm and a fluorescent dye having an excitation wavelength of approximately 630 nm (for example, Cy5).

Depending on the nucleic acid amplification method to be used, the kit of the present embodiment may further include one or more of a nucleotide triphosphate as a substrate, a nucleic acid synthesis enzyme, and a buffer solution for an amplification reaction. The nucleotide triphosphate is a substrate (dNTP, rNTP, or the like) for a nucleic acid synthesis enzyme. The nucleic acid synthesis enzyme is an enzyme for the nucleic acid amplification method to be used, and examples thereof include a DNA polymerase, an RNA polymerase, and a reverse transcriptase. Examples of the buffer solution for an amplification reaction include a tris buffer solution, a phosphate buffer solution, a veronal buffer solution, a boric acid buffer solution, and a Good's buffer solution, and the pH is not particularly limited.

### [Other Configurations]

The kit of the present embodiment may further include a buffer solution. The buffer solution can be appropriately selected from those known in the field, and examples thereof include a phosphate buffer solution, a tris hydrochloride buffer solution, a citrate buffer solution, a carbonate buffer solution, a borate buffer solution, a succinate buffer solution, and an acetate buffer solution. The buffer solution may include at least one of NaCl, a surfactant (for example, Tween 20 and Triton X-100), or a preservative (for example, sodium azide), as necessary. Specific examples of the buffer solution include phosphate buffered saline (PBS), PBS-Tween 20 (PBST), tris buffered saline (TBS), and TBS-Tween 20 (TBS-T).

In the kit of the present embodiment, the substance (specific antibody) that specifically binds to CD86, the primer set, and the like may be in a dry state or may be dissolved in the above-described buffer solution. Among those, the dry state is preferable from the viewpoint of storage stability.

### <Other Embodiments>

In one embodiment, the present invention provides a biomarker consisting of CD86 for predicting or diagnosing the efficacy of a treatment with immunotherapeutic agent for a patient with mucosal lesion.

In one embodiment, the present invention provides a method for diagnosing the efficacy of a treatment with immunotherapeutic agent on a patient with mucosal lesion, that is, a companion diagnosis method for an immunotherapeutic agent for a patient with mucosal lesion, the method including:
a step of measuring an expression level of CD86 in a biological sample derived from the patient with mucosal lesion.

In one embodiment, the present invention provides a use of a substance that specifically binds to CD86, or a forward primer and a reverse primer for amplifying a nucleic acid that encodes CD86, for manufacturing a kit for predicting the efficacy of a treatment with immunotherapeutic agent on a patient with mucosal lesion. Examples of the substance that specifically binds to CD86 include the same ones as described above, and the anti-CD86 antibody is preferable.

In one embodiment, the present invention provides immunotherapeutic agent to be administered to a patient having CD86 expression level equal to or less than a reference value, in which the immunotherapeutic agent is a drug that induces anti-HPV cellular immunity, and has a polypeptide derived from E7 protein of HPV as a drug that induces the anti-HPV cellular immunity. Examples of the polypeptide derived from the E7 protein of HPV include the same ones as described above. The mutated E7 protein in which amino acid residues involved in the binding of the E7 protein to the Rb protein correspond to Asp at position 21, Cys at position 24, and Glu at position 26 in the wild-type E7 protein (SEQ ID NO: 1) of HPV16 is preferable, and the mutated E7 protein (SEQ ID NO: 2) in which all of Asp at position 21, Cys at position 24, and Glu at position 26 in the wild-type E7 protein (SEQ ID NO: 1) of HPV16 are substituted with Gly is more preferable.

The present disclosure can include the following aspects.

A method for treating a patient with mucosal lesion with immunotherapeutic agent, the method including a step of measuring an expression level of CD86 in a biological sample derived from the patient with mucosal lesion; and (a) a step of administering an immunotherapeutic agent to a patient with mucosal lesion, based on evaluation results of an expression level of CD86 in a case where the expression level of CD86 is equal to or less than a preset reference value, or (b) a step of administering a drug that induces anti-HPV cellular immunity in combination with a CTLA-4 inhibitor that is an immune checkpoint inhibitor to a patient with a mucosal lesion in a case where the mucosal lesion is HPV infection or HPV-related tumor, the administered immunotherapeutic agent is the drug that induces anti-HPV cellular immunity, and the expression level of CD86 is more than a preset reference value.

### Examples

The present invention will be described below with reference to Examples, but is not limited to the following Examples.

### [Example 1]

### (Selection of Biomarker)

Antigen-presenting cells (cancer cells), and immune checkpoint molecules and cell surface markers expressed in various T cells were used as candidates for a biomarker indicating an immunological environment in the local cervix. Specifically, 3 types of factors of PD-L1, CD80, and CD86 were specified as factors on the cancer cell side, and 4 types of factors of Foxp3, PD-1, CD8, and CD4 were specified as factors on the T cell side.

Next, the expression levels of these 7 factors and the HPV16 E7 protein which is a target of the immune response in the exfoliated cervical cells were measured by a quantitative RT-PCR method.

Specifically, first, among patients with cervical intraepithelial neoplasia (CIN) which is a precancerous lesion of cervical cancer, 21 patients including patients with moderate dysplasia (CIN2) and patients with severe dysplasia/carcinoma in situ (CIN3) were administered immunotherapeutic agent at a dose of 1.5 g/patient, once a day before breakfast, for 5 days, in 4 cycles during weeks 1, 2, 4, and 8.

An immunotherapeutic agent (hereinafter also referred to as "IGMKK16E7") was prepared using the method described in Japanese Patent No. 6464201. Specifically, a lactic acid bacterium Lacticaseibacillus paracasei strain, which had been caused to express the E7 protein (full length) of the HPV16 type on the bacterial cell surface, was killed and used as an active pharmaceutical ingredient of IGMKK16E7. A freeze-dried powder of the bacterial cell that had been subjected to the killing treatment was encapsulated in a seamless capsule. One stick was packaged with 0.5 g of the IGMKK16E7 active pharmaceutical ingredient (that is, the patient was instructed to take 3 sticks per dose).

At the end of the administration period of the immunotherapeutic agent, the number of patients who were normal (CR) or had mild dysplasia (CIN1) (PR) and the number of patients who had no change in lesions (SD) were counted. The proportion of the number of the CR and PR patients to the total number of the patients who had been subjected to the administration test (CR/PR rate) was calculated.

At the end of the administration period of the immunotherapeutic agent, the exfoliated cervical cells of each patient were collected as samples using liquid-based cytology ThinPrep (registered trademark) (manufactured by Hologic Japan, Inc.). Total RNA was recovered from each sample using miRNeasy Mini Kit (217004, manufactured by QIAGEN N. V.). cDNA was synthesized from the recovered total RNA using a High-Capacity cDNA Reverse Transcription Kit (4368814, manufactured by Thermo Fisher Scientific Inc.), 7 candidate factors were amplified using the primers and probes shown below, and the expression levels were measured.

The sequences of forward primers, reverse primers, and probes used for detection of HPV16E7 and PD-1 are shown in Table 1. For the detection of Foxp3, CD8, CD4, CD80, CD86, and PD-L1, a pre-design qPCR probe purchased from Thermo Fisher Scientific Inc. was used. The Assay ID of each probe is shown in Table 2.

**[Table 1]**

| Type | | Base sequence (5' → 3') | SEQ ID NO |
|---|---|---|---|
| HPV16E7 | Forward primer | AAGTGTGACTCTACGCTTCGGTT | 3 |
| | Reverse primer | GCCCATTAACAGGTCTTCCAAA | 4 |
| | Probe | AGGAGCGACCCGGAAAGTTACCACAGTT | 5 |
| PD-1 | Forward primer | AGGCATGCAGATCCCACA | 6 |
| | Reverse primer | CCTGTCTGGGGAGTCTAAGA | 7 |
| | Probe | TCTGGGCGGTGCTACAACT | 8 |

**[Table 2]**

| Type | Assay ID |
|---|---|
| Foxp3 | Hs01085834_m1 |
| CD8 | Hs00233520_m1 |
| CD4 | Hs01058407_m1 |
| CD80 | Hs01045161_m1 |
| CD86 | Hs01567026_m1 |
| CD274 (PD-L1) | Hs00204257_m1 |

As a positive control, the E7-specific immune response ability (E7-CMI) in the peripheral blood was analyzed before (Pre) and after (16w/24w or Post) administration of the immunotherapeutic agent. In E7-CMI-16/24w, it was shown that the immune response ability is significantly high in the CR/PR case, and it could be confirmed that the immune response (E7-CMI) induced by oral administration of the immunotherapeutic agent correlates with the clinical therapeutic effect.

The results of the ROC analysis for each factor are shown in Table 3 below.

**[Table 3]**

| | | | 95% Confidence interval | | χ² | P value |
|---|---|---|---|---|---|---|
| | AUC | Standard deviation (SD) | Lower limit value | Upper limit value | | |
| E7-CMI-Pre | 0.6667 | 0.1350 | 0.4021 | 0.9313 | 1.5241 | 0.2170 |
| E7-CMI-16w/24w | 0.7593 | 0.1216 | 0.5210 | 0.9975 | 4.5479 | 0.0330 |
| Foxp3 | 0.5741 | 0.1319 | 0.3155 | 0.8327 | 0.3152 | 0.5745 |
| PD-L1 | 0.5370 | 0.1339 | 0.2747 | 0.7994 | 0.0766 | 0.7820 |
| PD-1 | 0.6296 | 0.1320 | 0.3708 | 0.8884 | 0.9639 | 0.3262 |
| CD8 | 0.6204 | 0.1298 | 0.3660 | 0.8748 | 0.8600 | 0.3538 |
| CD4 | 0.5833 | 0.1309 | 0.3267 | 0.8399 | 0.4052 | 0.5244 |
| CD80 | 0.5741 | 0.1368 | 0.3059 | 0.8423 | 0.2930 | 0.5883 |
| CD86 | 0.7963 | 0.0991 | 0.6021 | 0.9905 | 8.9432 | 0.0028 |
| HPV16E7 | 0.5093 | 0.1363 | 0.2421 | 0.7765 | 0.0046 | 0.9458 |

As shown in Table 3 above, the factors having a significant correlation with the CR/PR cases were only the E7-specific immune response ability and CD86 after the administration of the immunotherapeutic agent. In addition, a threshold value was set from the ROC curve, the results were divided into High and Low at the threshold value, and the number of CR/PR cases and the number of SD cases were subjected to a significance test using a χ² test. As a result, only CD86 was identified as a biomarker capable of selecting the CR/PR cases.

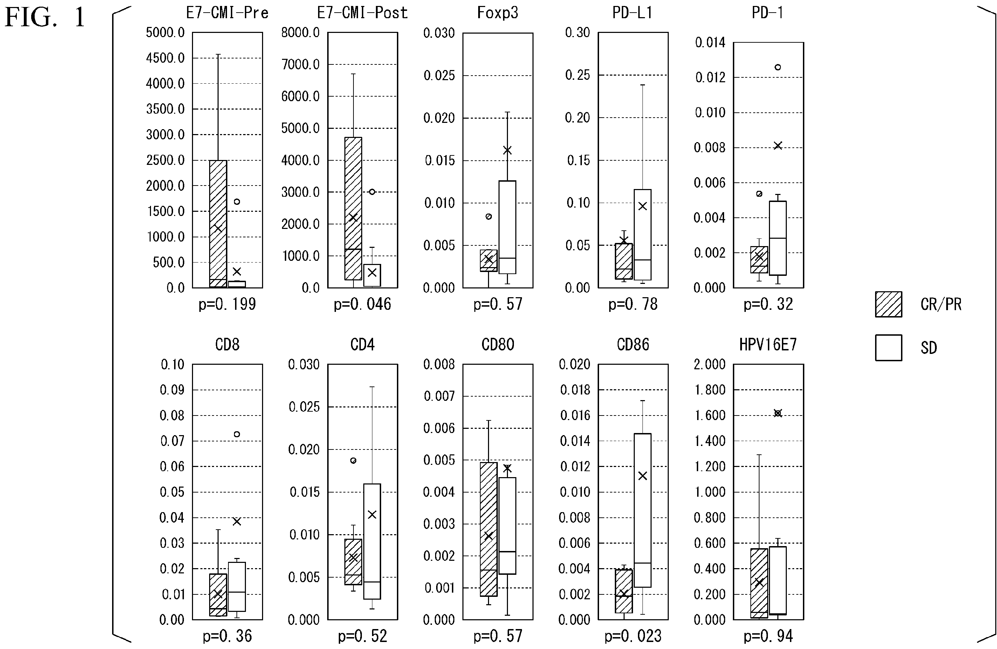

FIG. 1 is a graph showing the expression level of each factor in CR/PR cases and SD cases by an RT-PCR method. As shown in FIG. 1, for the E7-CMI-Post, the value was significantly high in the CR/PR case. Among the 7 biomarker candidates indicating the local immunological environment of the cervix, only CD86 had a significantly low value in the CR/PR case.

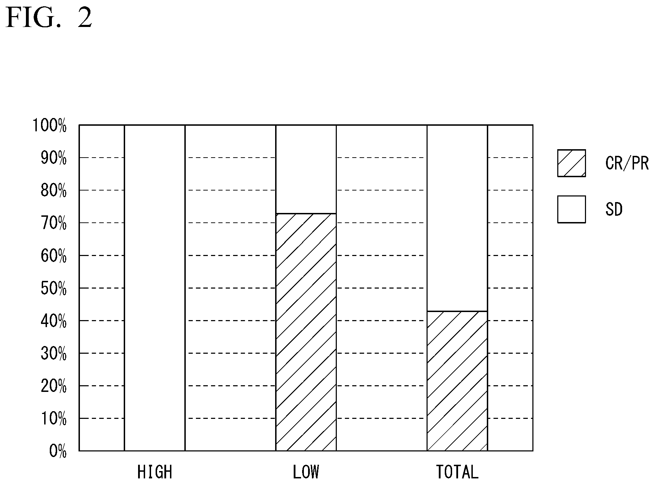

A graph showing the CR/PR rates in the total IGMKK16E7 administration group, CD86^{high} cases, and CD86^{low} cases is shown in FIG. 2. As shown in FIG. 2, the CR/PR rate in the total IGMKK16E7 administration group was 45% (9/21).

In the CD86^{high} case, the CR/PR rate was 0% (0/10). On the other hand, in the CD86^{low} cases, the CR/PR rate was 73% (8/11), and the significant difference was p = 0.001.

From the above results, it has been shown that the CR/PR rate of an immunotherapeutic agent can be improved by using the expression level of CD86 in the companion diagnosis for an immunotherapeutic agent.

### [Example 2]

### (Selection of Biomarker)

Antigen-presenting cells (cancer cells), and immune checkpoint molecules and cell surface markers expressed in various T cells were used as candidates for a biomarker indicating an immunological environment in the local cervix. Specifically, 3 types of factors of PD-L1, CD80, and CD86 were specified as factors on the cancer cell side, 5 types of factors of Foxp3, PD-1, CTLA4, CD8, and CD4 were specified as factors on the T cell side, and CD103 was specified as a mucosal immune factor.

Next, the expression levels of these 9 factors and the HPV16 E7 protein which is a target of the immune response in the exfoliated cervical cells were measured by a quantitative RT-PCR method.

Specifically, first, the immunotherapeutic agent was administered to 42 CIN2 patients and CIN3 patients (IGMKK16E7 administration group) by the same method as the administration method in Example 1. On the other hand, placebo was administered to 34 CIN2 patients and CIN3 patients (placebo administration group).

As the immunotherapeutic agent, the same immunotherapeutic agent as that prepared in Example 1 was used.

At the end of the administration period of the immunotherapeutic agent, the number of patients who were normal (CR), the number of patients who had mild dysplasia (CIN1) (PR), and the number of patients who had no change in lesions (SD) were counted. The proportion of the number of the CR patients to the total number of the patients who had been subjected to the administration test (CR rate) was calculated.

At the end of the administration period of the immunotherapeutic agent, the expression levels of 9 candidate factors were measured by amplifying the 9 candidate factors using the following primers and probes in the same manner as in Example 1.

The sequences of the forward primer, the reverse primer, and the probe used for detection of HPV16E7 and PD-1 are the same as those shown in Table 1. For the detection of Foxp3, CD8, CD4, CD80, CD86, PD-L1, CTLA4, and CD103, a pre-design qPCR probe purchased from Thermo Fisher Scientific Inc. was used. The Assay IDs of the probes used for the detection of Foxp3, CD8, CD4, CD80, CD86, and PD-L1 are the same as those shown in Table 2. The Assay IDs of the probes used for detection of CTLA4 and CD103 are each shown in Table 4.

**[Table 4]**

| Type | Assay ID |
|---|---|
| CTLA4 | Hs00175480_m1 |
| CD103 | Hs01025372_m1 |

The results of the ROC analysis for each factor are shown in Table 5 below.

**[Table 5]**

| | AUC | Standard deviation (SD) | 95% Confidence interval | | Odds ratio | P value |
|---|---|---|---|---|---|---|
| | | | Lower limit value | Upper limit value | | |
| Foxp3 | 0.60 | 0.092 | 0.42 | 0.78 | 2.3 | 0.290 |
| PD-L1 | 0.52 | 0.098 | 0.33 | 0.71 | 1.6 | 0.820 |
| PD-1 | 0.65 | 0.097 | 0.46 | 0.84 | 6.8 | 0.130 |
| CD8 | 0.56 | 0.100 | 0.36 | 0.76 | 3.1 | 0.560 |
| CD4 | 0.51 | 0.090 | 0.33 | 0.68 | 2.1 | 0.940 |
| CD80 | 0.57 | 0.100 | 0.37 | 0.77 | 3.0 | 0.510 |
| CD86 | 0.71 | 0.089 | 0.53 | 0.88 | 4.2 | 0.020 |
| CTLA4 | 0.62 | 0.093 | 0.43 | 0.80 | 3.6 | 0.210 |
| CD103 | 0.66 | 0.094 | 0.48 | 0.85 | 5.0 | 0.084 |
| HPV16 E7 | 0.61 | 0.089 | 0.43 | 0.78 | 3.5 | 0.230 |

As shown in Table 5, the factor having a significant correlation with the CR case was only CD86. In addition, a threshold value was set from the ROC curve, the results were divided into High and Low at the threshold value, and the number of CR cases and the number of PR/SD (non-CR) cases were subjected to a significance test using a χ² test. As a result, only CD86 was identified as a biomarker capable of selecting the CR cases.

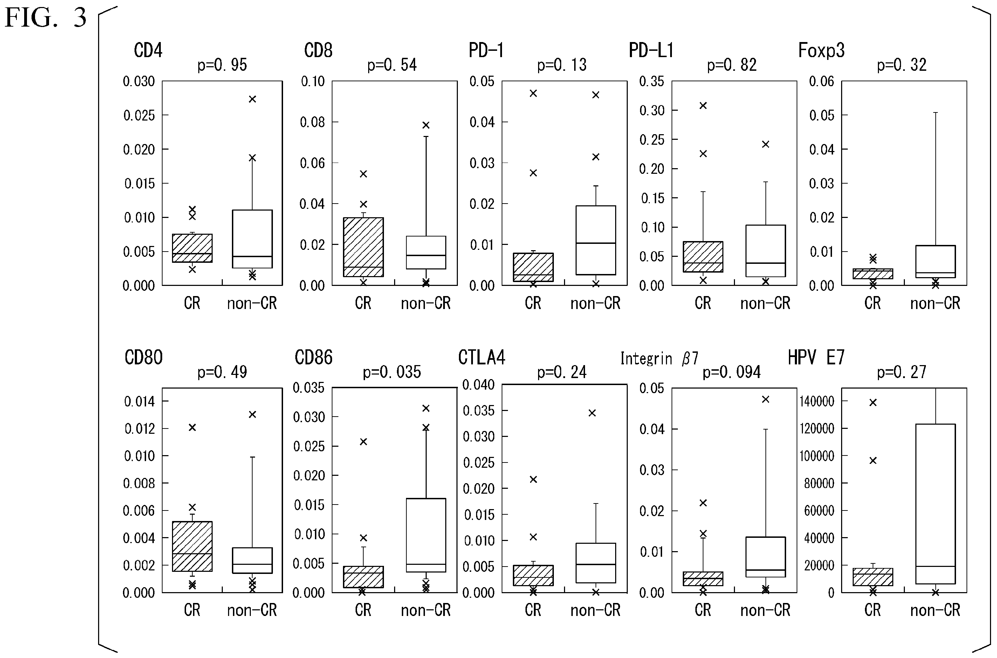

FIG. 3 is a graph showing the expression level of each factor in the CR cases and the non-CR cases by an RT-PCR method. Among the 10 biomarker candidates indicating the immunological environment of the cervix, only CD86 had a significantly low value in the CR case (p = 0.035).

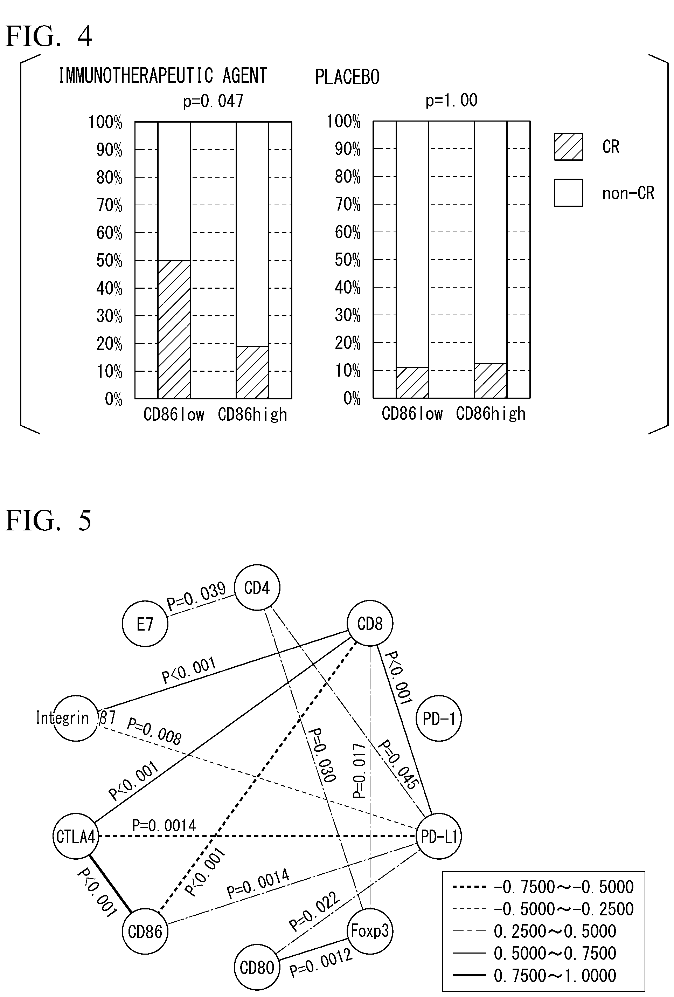

A graph showing the CR rates in the CD86^{high} case and the CD86^{low} case in the IGMKK16E7 administration group, and the CR rates in the CD86^{high} case and the CD86^{low} case in the placebo administration group are shown in FIG. 4. As shown in FIG. 4, the CR rate in the total IGMKK16E7 administration group was 31% (13/42). In the CD86^{high} case, the CR rate was 19% (5/26). On the other hand, in the CD86^{low} case, the CR rate was 50% (8/16) and the significant difference was p = 0.047. On the other hand, in the placebo administration group, the CR rate was 11% or 13% (2/16, 2/18) regardless of the CD86^{high} case and the CD86^{low} case, and there was no difference (p = 1.00). The difference in efficacy depending on the expression level of CD86 was a phenomenon specific to the IGMKK16E7 administration group.

FIG. 5 is a graph showing a partial correlation matrix between the expression level of CD86 and other factors correlated therewith. A solid line in the figure indicates a positive correlation and a broken line indicates a negative correlation. CD86 had a strong positive correlation with CTLA4 and a strong negative correlation with CD8. In the local immunological environment of the cervix, high expression of CD86 reflects high expression of CTLA4 and low expression of CD8. Since the CTLA4/CD86 pathway is a pathway that suppresses the activity of T cells and CD8 is a marker for cytotoxic T cells among T cells, it reflects that the local T cells in the cervix in the CD86^{high} case are in a suppressed state.

From the results above, it has been shown that the CR rate of an immunotherapeutic agent can be improved by using the expression level of CD86 in the companion diagnosis for the immunotherapeutic agent.

### INDUSTRIAL APPLICABILITY

By the method and the kit of the present embodiment, the efficacy of a treatment with an immunotherapeutic agent on a patient with a mucosal lesion can be predicted.

While preferred embodiments of the present invention have been described and illustrated above, it should be understood that these are exemplary of the present invention and are not to be considered as limiting. Additions, omissions, substitutions, and other changes can be made without departing from the spirit or scope of the present invention. Accordingly, the present invention is not to be considered as being limited by the foregoing description, and is only limited by the scope of the appended claims.

## Claims

1. A method for predicting efficacy of a treatment with immunotherapeutic agent on a patient with mucosal lesion, the method comprising:
a step of measuring an expression level of CD86 in a biological sample derived from the patient with mucosal lesion.

2. The method according to Claim 1, further comprising:
a step of evaluating or predicting that the treatment with immunotherapeutic agent is potentially efficacious in a case where the expression level of CD86 is equal to or less than a preset reference value.

3. The method according to Claim 1,
wherein the mucosal lesion is human papillomavirus infection or human papillomavirus-related tumor.

4. The method according to Claim 3,
wherein the human papillomavirus-related tumor is cervical intraepithelial neoplasia.

5. The method according to Claim 4,
wherein the biological sample is exfoliated cervical cells.

6. The method according to Claim 3,
wherein the immunotherapeutic agent is a drug that induces anti-human papillomavirus cellular immunity.

7. A kit for use in prediction of efficacy of a treatment with immunotherapeutic agent on a patient with mucosal lesion, the kit comprising:
a substance that specifically binds to CD86, or a forward primer and a reverse primer for amplifying a nucleic acid that encodes CD86.

8. A kit for use in the method according to any one of Claims 1 to 6, the kit comprising:
a substance that specifically binds to CD86, or a forward primer and a reverse primer for amplifying a nucleic acid that encodes CD86.

9. The kit according to Claim 8,
wherein the substance that specifically binds to CD86 is anti-CD86 antibody.
